# EUROPEAN PATENT SPECIFICATION

(11) **EP 1 474 186 B1**
(45) Date of publication and mention of the grant of the patent: **31.05.2006**
(21) Application number: 02777440.5
(22) Date of filing: 21.10.2002
(51) Int. Cl.: A61M 1/00

(54) **INFANT NASAL ASPIRATOR**
NASENABSAUGVORRICHTUNG FÜR KLEINKINDER
ASPIRATEUR NASAL POUR NOURRISSON

(30) Priority: 22.10.2001 GB 0125269; 19.11.2001 GB 0127656
(43) Date of publication of application: 10.11.2004
(73) Proprietor: MORETTO, Carolyn, Derbys DE75 7TW (GB)
(72) Inventor: MORETTO, Carolyn, Derbys DE75 7TW (GB)
(74) Representative: Powell, Timothy John
(86) International application number: PCT/GB2002/004741
(87) International publication number: WO 2003/035144

(56) References cited:
- EP-A- 0 451 062
- WO-A-99/53819
- GB-A- 2 234 902
- US-A- 5 098 418

## Description

The present invention relates to a nasal aspirator for infants intended to help relieve blocked noses.

### Background of the Invention

Up to about two years of age when they acquire the ability to blow their noses, infants require assistance with clearing nasal congestion. Since colds and flu are very common among the young parents find themselves regularly clearing their children's noses.

Aspirators for clearing congestion in infants are found in hospitals and in the domestic environment. Aspirators used in hospitals are generally electric machines that while effective are too expensive for application in the household Less expensive domestic aspirators are usnally hand-beld and 'pear' shaped with a pump. One of the dangers with these is that since they lack a one-way valves there is a possibility of the congestion being pushed further up the nasal cavity rather than being.sucked out.

Additionally the pump makes it difficult for the user to know whether the squeezing action is extracting mucus or pushing it in further.

The present aspirator provides a safe and hygienic means of relieving congestion in infants. It is as effective as an electric aspirator and as affordable as domestic aspirators presently on the market.

EP 0 431 062 discloses an infant nasal aspirator comprising a flexible tube attached to one end to a suction means (ball member) and with the other end in communication with an end piece having a through-going opening and tapering from a rear end to a nozzle and a filter located inside the end piece. However, EP 0 451 062 does not provide that said suction means is a mouthpiece which is adapted to be inserted or placed against a user's mouth, and that the mouthpiece is a short pipe with a circular flange extending around the middle of the pipe dividing the pipe into a mouth section and a tube section.

### Summary of the Invention

According to the present invention there is provided an infant nasal aspirator for clearing nasal congestion in infants comprising a flexible tube attached at one end to a suction means and with the other end in communication with an end piece, the end piece having a through-going opening and tapering from a rear end to a nozzle which, in use, is positioned at the entrance of an infant's nostril; and a filter located inside the end piece, wherein the filter absorbs congestion sucked into the aspirator and prevents the congestion from entering the tube or returning to the infant's nostril.

The suction means is a mouthpiece which is adapted to be inserted or placed against a user's mouth for the user to suck through to create a vacuum in the aspirator. The mouthpiece is a short pipe with a circular flange extending around the middle of the pipe dividing the pipe into a mouth section and a tube section.

Preferably, the end piece is transparent, such that the interior is visible; and disposable.

All the aspirator components, aside from the filter, are preferably made of plastics. The filter is preferably made of sponge or gauze.

A hollow cylindrical cap may be located between the tube and the end piece, the tube being fitted onto a port at one end of the cap and the end piece fitting onto an opening at the other end of the cap.

### Brief Description of the Drawings

There now follows a description of preferred embodiments of the invention, by way of non-limiting example, with reference being made to the accompanying drawings in which:
Figure 1 is a schematic view of an infant nasal aspirator according to a first embodiment of the invention;
Figure 2 schematically illustrates a hand pump for an aspirator and
Figure 3 is a cross-sectional view of the first embodiment of the aspirator showing a different mouthpiece but no tube or filter.

### Detailed Description of Preferred Embodiments of Invention

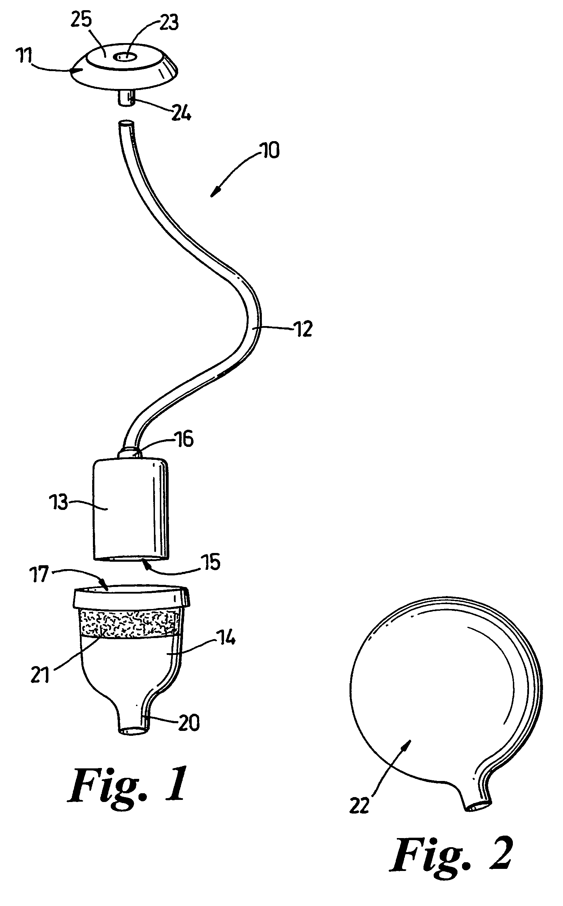

A first embodiment of an infant nasal aspirator 10 as illustrated in Figure 1 comprises a hollow mouthpiece 11 connected to one end of a hollow tube 12. The other end of the tube 12 is connected to a port 16 in a first end of a hollow cylindrical cap 13. An end piece 14 having a nozzle 20 is removably fitted onto an opening 15 at a second end of the cap 13. A filter 21 sits inside a through-going opening 17 of the end piece and generally towards the rear, that is, at the end opposite the nozzle 20.

The above components of the aspirator are all detachably connected with a secure fit and, aside from the filter, are made of plastics. The filter is made of sponge or gauze, such as muslin. The tube 12 is made of a flexible plastic to allow relative movement of the mouthpiece and cap. The end piece 14 is disposable for hygiene purposes but is made of a plastic sufficiently hardy to withstand several washes and/or treatment in a steriliser for re-use if desired.

The mouthpiece 11 in the embodiment of Figure 1 is a round flange containing a through-hole 23 through its centre for the passage of air. The tube is securely fitted on a mouthpiece port 24 in communication with the hole 23. The user's lips are pressed against the flat 25 of the mouthpiece to cover hole 23 such that the user can draw air through the tube and mouthpiece.

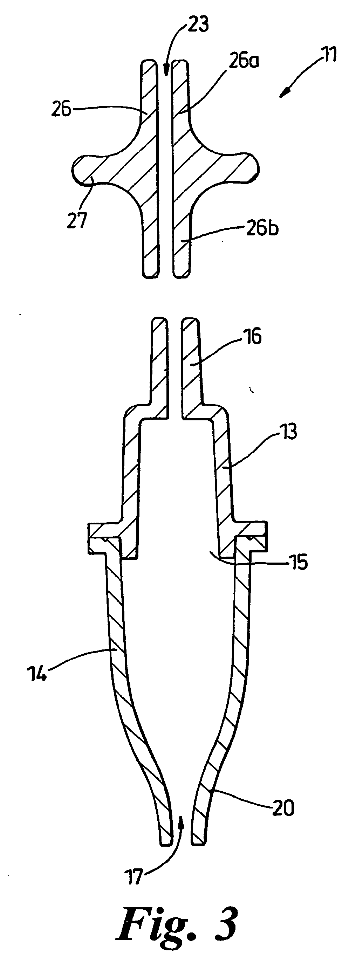

In the embodiment shown in Figure 3 the mouthpiece is a short pipe 26 with a circular flange 27 extending around the middle of the pipe. Flange 27 divides the pipe into two sections: a mouth section 26a for a user to take into the mouth; and a tube section 26b over which the tube can be fitted and securely connected to the mouthpiece.

The end piece 14 in Figure 3 is circular in cross section and tapers from its larger rear end, at which the cap 13 is attached, to the smaller cross section of the nozzle. The filter occupies the entire cross sectional area inside the through-going opening 17 and sits generally towards the rear end.

The aspirator is adapted to clear mucus from the noses of infants. With all components connected one to the other the nozzle 20 is gently placed at the entrance of one nostril. The mouthpiece 11 is then placed into or against the user's mouth while the cap 13 is held steady. By sucking through the mouthpiece 11 the user creates a partial vacuum in the tube 12, cap 13 and end piece 14 thereby causing mucus in the nostril to be drawn into the aspirator through the nozzle.

The filter catches and absorbs the mucus and prevents it from entering the cap 13, the tube 12, the mouthpiece 11 and accordingly the user's mouth. By catching the mucus the filter acts as a differential valve allowing the mucus to be drawn in one direction only. It also acts as a true filter by providing a barrier between the user's mouth and the infant's nostril.

The end piece 14 is made of transparent plastic to allow a user to observe the mucus being sucked into the aspirator. The user can thereby be assured that the mucus is being drawn in the correct direction.

It logically follows that the user can know when the aspirator is full of mucus because not only is it visible through the end piece but the filter full of mucus creates a stop in the suction line which is detected by the user.

The use of mouth suction generates "feel" or control in the mucus withdrawing operation. This benefit is unavailable in prior art aspirators as described hereinabove.

The mouthpiece 11 may be replaced with a plastic hand operated pump 22, as shown in Figure 2, to draw air through the aspirator. The pump 22 is a round flexible bellows having a one-way valve that prevents it from dangerously pumping air in the wrong direction into the aspirator.

In this case the end piece is placed in the entrance of the infant's nostril in the same manner as with the first embodiment. To draw mucus the user squeezes the pump to create a vacuum through the aspirator. Since the end piece is clear the filter housed inside is visible and so too is mucus drawn from the infant's nostril. The user stops squeezing the pump when it is evident that congestion has cleared.

If the filter absorbs its capacity of mucus before congestion is cleared, the filter is simply disposed of and replaced with a clean filter, or, alternatively, the entire end piece is replaced with a clean one.

The infant nasal aspirator provides a barrier between the user and infant allowing only air to pass until the filter is satiated. Owing to the one-way valve function of the filter, there is no possibility of mucus being pushed back into the nostril regardless of how irregularly or hard the pump is squeezed, or how hard the user sucks on the mouthpiece.

The infant nasal aspirator effectively clears nasal congestion in infants without discomfort to the infant or the user. The aspirator will be affordable in the domestic market and an effective means of relief for congested infants.

In the embodiment shown the various detachable plastics parts of the aspirator are securable together by means of interengageable, "push-fit" components eg. constituted by ribs and recesses formed respectively in mating components. Such arrangements are well known in the art.

Other methods of securing the detachable plastics parts together are within the scope of the invention. For example, mutually complementary screw threads may be employed in the parts requiring connection. Numerous equivalent arrangements are also possible within the scope of the invention.

With reference to the use in the apparatus of the invention of a disposable filter or filter-containing cartridge or chamber in a suction-actuated nasal aspirator, the filter or filter-containing cartridge preferably is or includes a sponge- or gauze-like material as disclosed herein that when partly charged with mucus acts as a one way valve. When the filter is completely blocked it provides an automatic signal of the time to change the filter or cartridge. when the cartridge is used as part of the embodiment of Figure 1 hereof.

## Claims

1. An infant nasal aspirator (10) for clearing nasal congestion in infants comprising a flexible to be (12) attached at one end to a suction means and with the other end in communication with an end piece (14), the end piece (14) having a through-going opening (15) and tapering from a rear end to a nozzle (20) which, in use, is positioned at the entrance an infant's nostril; and filter (21) located inside the end piece (16), wherein the filter (21) absorbs congestion sucked into the aspirator and prevents the congestion from entering the tube (12) or returning to the infant's nostril, **characterised in that** the suction means is a mouthpiece (11) which is adapted to be inserted or placed against a user's mouth for the user to suck through to create a vacumm in the aspirator, and wherein the mouthpiece (11) is a short pipe with a circular flange extending around the middle of the pipe dividing the pipe into a mouth section and a tube section.

2. The infant nasal aspirator (10) claimed in any one of the preceding claims wherein the end piece (14) is transparent such that the interior is visible.

3. The infant nasal aspirator (10) claimed in any one of the preceding claims wherein the filter (21) is made of sponge or ganze.

4. The infant nasal aspirator (10) clamied in any one of the preceding claims wherein a hollow cylindrical cap (13) is located between the tube (12) and the end piece(14), the tube (12) being fined onto a port (16) at one end of the cap (13) and the and piece (14) fitting onto an opening at the other end of the cap (13).

5. The infant nasal aspirator (10) claimed in any one of the preceding claims wherein the end piece (14) is disposable.

6. The infant nasal aspirator (10) claimed in any one of the preceding claims wherein all the aspirator components, aside from the filter (21), are made of plastics.

7. The infant nasal aspirator (10) as claimed in any of the preceding claims, the end piece (14) of which is made from a transparent plastic.

## Patentansprüche

1. Nasenabsaugvorrichtung (10) für Kleinkinder zum Entfernen von in der Nase eines Kleinkindes angesammelten Substanzen, die: einen flexiblen Schlauch (12) umfasst, der an einem Ende mit einer Saugeinrichtung verbunden ist und am anderen Ende mit einem Endstück (14) in Verbindung steht, wobei das Endstück (14) eine durchgehende Öffnung (15) aufweist und sich von einem hinteren Ende zu einer Düse (20) hin verjüngt, die bei Benutzung am Eingang eines Nasenlochs eines Kleinkindes angeordnet wird, wobei ein Filter (21) innerhalb des Endstückes (14) angeordnet ist und der Filter (21) angesammeltes Material absorbiert, das in die Absaugvorrichtung gesaugt wird, und verhindert, dass das angesammelte Material in den Schlauch (12) eintritt oder in das Nasenloch des Kleinkindes zurückkehrt, **dadurch gekennzeichnet, dass** die Saugeinrichtung ein Mundstück (11) ist, das geeignet ist, in den Mund eines Verwenders eingeführt oder am Mund eines Verwenders angeordnet zu werden, damit der Verwender an ihm saugen kann, um einen Unterdruck in der Absaugvorrichtung zu erzeugen, und dass das Mundstück (11) ein kurzes Rohr mit einem kreisförmigen Flansch ist, der sich um die Mitte des Rohrs herum erstreckt und das Rohr in einen Mundabschnitt und in einen Schlauchabschnitt unterteilt.

2. Nasenabsaugvorrichtung (10) für Kleinkinder nach dem vorhergehenden Anspruch, bei der das Endstück (14) durchsichtig ist, so dass sein Inneres sichtbar ist.

3. Nasenabsaugvorrichtung (10) für Kleinkinder nach einem der vorhergehenden Ansprüche, bei der das Filter (21) aus einem Schwamm oder aus Gaze besteht.

4. Nasenabsaugvorrichtung (10) für Kleinkinder nach einem der vorhergehenden Ansprüche, bei der eine hohle zylindrische Kappe (13) zwischen dem Schlauch (12) und dem Endstück (14) angeordnet ist, wobei der Schlauch (12) auf einen Durchgang (16) an einem Ende der Kappe (13) aufgepasst ist und das Endstück (14) auf eine Öffnung am anderen Ende der Kappe (13) passt.

5. Nasenabsaugvorrichtung (10) für Kleinkinder nach einem der vorhergehenden Ansprüche, bei der das Endstück (14) wegwerfbar ist.

6. Nasenabsaugvorrichtung (10) für Kleinkinder nach einem der vorhergehenden Ansprüche, bei der alle Bestandteile der Absaugvorrichtung mit Ausnahme des Filters (21) aus Kunststoff hergestellt sind.

7. Nasenabsaugvorrichtung (10) für Kleinkinder nach einem der vorhergehenden Ansprüche, deren Endstück (14) aus durchsichtigem Kunststoff hergestellt ist.

## Revendications

1. Aspirateur nasal pour enfant (10) servant à éliminer les sécrétions nasales chez l'enfant, comprenant un tube flexible (12) fixé par une extrémité à un dispositif d'aspiration et l'autre extrémité communiquant avec un élément terminal (14), l'élément terminal (14) présentant une ouverture (15) au travers de celui-ci et se rétrécissant de l'extrémité arrière jusqu'à une embouchure (20) qui, lors de l'utilisation, est placée à l'entrée de la narine d'un enfant ; et un filtre (21) situé à l'intérieur de l'élément terminal (16) ; dans lequel le filtre (21) absorbe les sécrétions aspirées dans l'aspirateur et empêche celles-ci d'entrer dans le tube (12) ou de retourner dans la narine de l'enfant, **caractérisé en ce que** le dispositif d'aspiration est un élément buccal (11) qui est adapté de façon à être inséré dans la bouche d'un utilisateur ou contre celle-ci pour que l'utilisateur exerce une aspiration afin de créer un vide dans l'aspirateur ; et dans lequel l'élément buccal (11) est un tuyau court doté d'un bord circulaire s'étendant autour du milieu du tuyau, divisant le tuyau en une partie buccale et une partie de tube.

2. Aspirateur nasal pour enfant (10) selon la revendication précédente, dans lequel l'élément terminal (14) est transparent, de telle sorte que l'intérieur soit visible.

3. Aspirateur nasal pour enfant (10) selon l'une quelconque des revendications précédentes, dans lequel le filtre (21) est constitué d'une éponge ou d'une gaze.

4. Aspirateur nasal pour enfant (10) selon l'une quelconque des revendications précédentes, dans lequel un embout cylindrique creux (13) est situé entre le tube (12) et l'élément terminal (14), le tube (12) étant fixé sur un orifice (16) à une extrémité de l'embout (13) et l'élément terminal (14) s'ajustant sur une ouverture à l'autre extrémité de l'embout (13).

5. Aspirateur nasal pour enfant (10) selon l'une quelconque des revendications précédentes, dans lequel l'élément terminal (14) est jetable.

6. Aspirateur nasal pour enfant (10) selon l'une quelconque des revendications précédentes, dans lequel tous les éléments de l'aspirateur, à l'exception du filtre (21), sont constitués de matières plastiques.

7. Aspirateur nasal pour enfant (10) selon l'une quelconque des revendications précédentes, dont l'élément terminal (14) est constitué de plastique transparent ;
